# FASCICULE DE BREVET EUROPEEN

(11) **EP 0 987 014 B1**
(45) Date de publication et mention de la délivrance du brevet: **31.05.2006**
(21) Numéro de dépôt: 99402069.1
(22) Date de dépôt: 16.08.1999
(51) Int. Cl.: A61K 8/06, A61K 8/81, A61Q 1/14, A61Q 19/00

(54) **Emulsion comprenant un composé épaississant hydrophile et un copolymère épaississant, compositions comprenant ladite émulsion, et utilisations**
Verdickungsmittel und verdickendes Copolymer enthaltende hydrophile Emulsion, diese enthaltende Zusammensetzungen und ihre Verwendung
Emulsion containing an hydrophile thickening agent and a thickening coplymer, compositions containing them and their uses

(30) Priorité: 16.09.1998 FR 9811577
(43) Date de publication de la demande: 22.03.2000
(73) Titulaire: L'OREAL, 75008 Paris (FR)
(72) Inventeur: Lorant, Raluca, 94320 Thiais (FR)
(74) Mandataire: Dodin, Catherine

(56) Documents cités:
- EP-A- 0 406 042
- EP-A- 0 482 417
- EP-A- 0 832 645

## Description

La présente invention a trait à une composition se présentant sous la forme d'une émulsion, susceptible d'être utilisée dans les domaines cosmétique et dermatologique, notamment pour le soin ou le traitement de la peau du corps ou du visage, plus particulièrement pour le soin ou le traitement des peaux sèches et/ou sensibles. L'invention concerne également une composition cosmétique ou dermatologique comprenant une telle émulsion.

Les compositions cosmétiques ou dermatologiques actuelles se présentent le plus souvent sous la forme d'émulsion du type huile-dans-eau (c'est à dire un support constitué d'une phase continue dispersante aqueuse et d'une phase discontinue dispersée huileuse) ou d'émulsion du type eau-dans-huile (c'est à dire un support constitué d'une phase continue dispersante grasse et d'une phase discontinue dispersée aqueuse).
Les émulsions eau-dans-huile comportent donc une phase continue huileuse et permettent de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches.
Les émulsions huile-dans-eau, quant à elles, apportent sur la peau à l'application un toucher plus doux, moins gras et plus léger que les émulsions eau-dans-huile.

Les émulsions sont stabilisées généralement par incorporation de tensioactifs émulsionnants du type huile-dans-eau (H/E) ou du type eau-dans-huile (E/H) qui, grâce à leur structure amphiphile, se placent à l'interface huile/eau et stabilisent ainsi les gouttelettes dispersées. Il est généralement nécessaire d'introduire ces tensioactifs en une quantité importante, pouvant aller jusqu'à 10 % en poids par rapport au poids total de l'émulsion, pour obtenir une stabilité adéquate.
Or, ces tensioactifs amphiphiles, utilisés en grande quantité, peuvent se montrer irritants pour la peau, les yeux et/ou le cuir chevelu des utilisateurs. De plus, leur présence à de fortes concentrations peut conduire à des effets non cosmétiques tels qu'un toucher rêche, collant et/ou poisseux, ou une composition finale compacte et lourde. D'autre part, les tensioactifs doivent être choisis en fonction de la polarité des huiles et ne sont donc compatibles qu'avec un nombre limité d'huiles, limitant ainsi la variété des formulations.

Les formulateurs d'émulsions recherchent constamment à réduire la teneur en tensioactif afin d'améliorer l'innocuité des émulsions vis à vis de la peau, des yeux et/ou du cuir chevelu et améliorer leurs propriétés cosmétiques. La principale difficulté à laquelle ils sont généralement confrontés est d'obtenir des émulsions stables.

Il a ainsi été proposé, par la demande WO96/37180, une composition susceptible d'applications dans les domaines pharmaceutique et/ou cosmétique, se présentant sous forme de 'pseudo-émulsion' et dépourvue de tensioactif. La composition comprend, d'une part, en phase aqueuse, un agent gélifiant choisi notamment parmi les polyoses ou les polymères acryliques, et d'autre part, en phase grasse, un facteur de consistance choisi parmi les corps gras cireux, et notamment les esters de glycérol. Le facteur de consistance présent dans la phase grasse est une substance semi-solide à 25°C et a un point de fusion supérieur à 50°C; il est solubilisé à chaud dans la phase grasse puis retrouve sa consistance semi-solide initiale à froid, conférant une certaine consistance et une certaine viscosité à ladite phase grasse. La composition ainsi obtenue présente une structure microscopique différente de celle d'une émulsion.
On a toutefois constaté qu'au delà d'une certaine quantité de phase grasse, ladite pseudo-émulsion perdait en stabilité, à la chaleur et/ou dans le temps. Ceci est en particulier vrai pour des taux de phase grasse supérieurs à 20% en poids par rapport au poids total de la pseudo-émulsion. Or, un taux de phase grasse important, dans une émulsion H/E, peut se révéler très intéressant, notamment pour les compositions cosmétiques destinées au soin des peaux sèches. De plus, les propriétés cosmétiques de cette pseudo-émulsion ne sont pas adéquates: sa texture est hétérogène, son toucher gras, et on constate une mauvaise prise sur le doigt.
Ainsi, il subsiste toujours le besoin de disposer d'une émulsion H/E qui soit stable au cours du temps bien qu'elle ne contienne pas de tensioactif, et qui puisse comprendre une quantité importante de phase grasse sans perdre sa stabilité.

La présente invention a pour but de pallier ce besoin et de proposer une émulsion, notamment une émulsion H/E ne présentant pas les inconvénients mentionnés ci-dessus, et qui soit stable tout en comprenant une quantité réduite de tensioactif, et qui puisse supporter une quantité importante de phase grasse.
Ainsi, la demanderesse a trouvé de manière surprenante qu'il était possible d'obtenir une émulsion ayant de bonnes propriétés cosmétiques et une bonne stabilité en utilisant une association particulière d'épaississants, de la phase huileuse et de la phase aqueuse.

La présente invention a donc pour objet une émulsion comprenant une phase aqueuse et une phase huileuse, et comprenant en outre au moins un composé épaississant hydrophile et au moins un copolymère épaississant comprenant au moins un motif hydrophobe dans une quantité suffisante pour obtenir sa solubilité partielle ou totale dans ladite phase huileuse, et au moins un motif hydrophile dans une quantité suffisante pour produire un épaississement de ladite phase huileuse; ledit motif hydrophile étant choisi parmi les monoacides carboxyliques à insaturation α,β-éthylénique en C₃-C₆, les diacides carboxyliques à insaturation α,β-éthylénique en C₄-C₆ ainsi que les dérivés monesters ou monoamides desdits diacides.

L'invention a également pour objet une composition cosmétique ou dermatologique comprenant, dans un milieu cosmétiquement ou dermatologiquement acceptable, une émulsion telle que définie ci-dessus.

L'invention a également pour objet l'utilisation d'une émulsion telle que définie ci-dessus, pour le traitement cosmétique de la peau, des cheveux, des cils, des sourcils, des ongles, des muqueuses, du cuir chevelu; en particulier pour le soin du visage et/ou du corps, le maquillage du visage et/ou du corps, le démaquillage; la protection solaire.
L'invention a également pour objet l'utilisation d'une telle émulsion pour la fabrication d'une composition dermatologique destinée au traitement de la peau, des cheveux, des cils, des sourcils, des ongles, du cuir chevelu et/ou des muqueuses. L'invention a également pour objet un procédé de traitement non thérapeutique de la peau, notamment des peaux sèches et/ou sensibles, des cheveux, des cils, des sourcils, des ongles, des muqueuses, du cuir chevelu, consistant à appliquer sur le support une telle émulsion ou une composition la comprenant.
L'invention a également pour objet l'utilisation d'un composé épaississant hydrophile et d'un copolymère épaississant tel que ci-dessus défini, pour stabiliser une émulsion, notamment une émulsion huile-dans-eau, et plus particulièrement une émulsion ne comprenant pas de tensioactif.

On a constaté que l'émulsion obtenue selon l'invention reste stable dans le temps à température ambiante ou à des températures plus élevées, malgré la faible quantité voire l'absence de tensioactif.
De plus, la phase grasse se disperse parfaitement dans la phase aqueuse et permet l'obtention d'une composition homogène, ayant la structure microscopique d'une émulsion.
L'émulsion obtenue est facile à appliquer sur la peau, les muqueuses, le cuir chevelu, les cheveux, les cils, les sourcils, les ongles.
Les textures obtenues sont particulièrement originales: les émulsions sont crémeuses et onctueuses, et n'ont absolument pas l'aspect gélifié, voire gélatineux, de certaines émulsions de l'art antérieur dont la phase aqueuse externe est épaissie.
Le toucher cosmétique sur la peau est également apprécié: à l'application, l'émulsion procure une sensation de fraîcheur et de confort, tout en étant riche et nourrissante; elle est douce et confortable et pas du tout collante.
L'émulsion ainsi obtenue est particulièrement adaptée pour le soin et le traitement des peaux sèches et/ou sensibles, de par la possibilité de la présence d'une quantité importante de corps gras apportant soin et confort, et de l'absence de tensioactif irritant.
De plus, les émulsions selon l'invention ne nécessitent pas de mode opératoire lourd et coûteux tel qu'une homogénéisation à haute pression, mais peuvent être préparées selon un mode opératoire classique.

L'émulsion selon l'invention se présente, de manière préférentielle, sous la forme d'une émulsion huile-dans-eau, comprenant une phase grasse ou huileuse interne dispersée dans une phase externe aqueuse.

La phase aqueuse peut comprendre de l'eau et/ou une eau thermale et/ou une eau de source et/ou une eau minérale et/ou une eau florale.
L'émulsion comprend par ailleurs au moins un composé épaississant hydrophile, qui peut être choisi parmi tous les épaississants hydrophiles connus de l'homme du métier. En particulier, on peut citer les composés suivants :
- les polymères synthétiques,
- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxy-méthylcellulose,
- le silicate de magnésium et d'aluminium;
- les épaississants inorganiques tels que les smectites, les hectorites modifiées ou non (BENTONE, LAPONITE par exemple),
- leurs mélanges.

Parmi les polymères synthétiques, on peut citer :
(A) les acides polyacryliques et notamment :
   (i) les polymères poly(méth)acrylates de glycéryl tels que l'HISPAGEL ou le LUBRAGEL des sociétés HISPANO QUIMICA ou GARDIAN,
   (ii) les homopolymères et les copolymères d'acide acrylique, éventuellement réticulés, ou un de leurs sels, tels que ceux commercialisés sous le nom 'CARBOPOL' par la société GOODRICH;
(B) les polymères à base de polyacrylamide et notamment :
   (i) le produit commercialisé sous le nom de SEPIGEL 305 par SEPPIC, et qui est constitué d'une émulsion H/E comprenant 35-45% en poids de copolymère réticulé d'acrylamide/acide 2-acrylamido 2-méthylpropane sulfonique neutralisé, 15-25% en poids d'hydrocarbures isoparaffiniques, 3-8% en poids de lauryléther de polyéthylèneglycol 70E, et d'eau;
   (ii) les copolymères acrylate/octyl-acrylamide tels que le DERMACRYL de National Starch;
   (iii) les polymères réticulés d'acrylamide et de chlorure de méthacryloyloxy éthyltriméthylammonium tels que le SALCARE SC92 de ALLIED COLLOIDS;
   (iv) les polymères réticulés d'acrylamide et d'acrylate d'ammonium tels que le PAS 5161 ou BOZEPOL C de HOECHST;
   (v) les polymères poly(acide 2-acrylamido 2-méthylpropane sulfonique) réticulés et pratiquement ou totalement neutralisés.
(C) les copolymères constitués d'une fraction majoritaire de monomère acide carboxylique monooléfiniquement insaturé en C₃-C₆ ou de son anhydride, et d'une fraction minoritaire de monomère ester à chaîne grasse d'acide acrylique, ces copolymères étant éventuellement réticulés.

Les polymères poly(acide 2-acrylamido 2-méthylpropane sulfonique) réticulés et pratiquement ou totalement neutralisés, susceptibles d'être utilisés dans le cadre de l'invention, sont hydrosolubles ou gonflables dans l'eau. Ils sont en général caractérisés par le fait qu'ils comprennent, distribués de façon aléatoire :
a) de 90 à 99,9% en poids de motifs de formule générale (1) suivante : 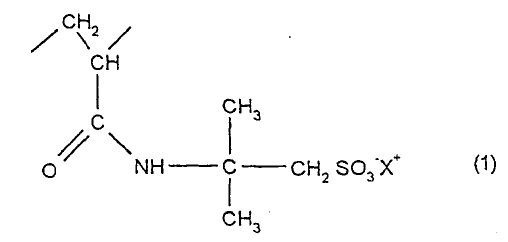 dans laquelle X⁺ désigne un cation ou un mélange de cations, au plus 10% mol des cations X⁺ pouvant être des protons H⁺ ;
b) de 0,01 à 10% en poids de motifs réticulants provenant d'au moins un monomère ayant au moins deux doubles liaisons oléfiniques ; les proportions en poids étant définis par rapport au poids total du polymère.
De façon préférentielle, ils comprennent de 98 à 99,5 % en poids de motifs de formule (1) et de 0,2 à 2 % en poids de motifs réticulants.
X⁺ représente un cation ou un mélange de cations choisis en particulier parmi un proton, un cation de métal alcalin, un cation équivalent de celui d'un métal alcalino-terreux ou l'ion ammonium. Plus particulièrement, 90 à 100% mole des cations sont des cations NH₄⁺ et 0 à 10% mole sont des protons (H⁺).
Les monomères de réticulation ayant au moins deux doubles liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.
Les monomères de réticulation ayant au moins deux doubles liaisons oléfiniques sont plus particulièrement choisis parmi ceux répondant à la formule générale (2) suivante : 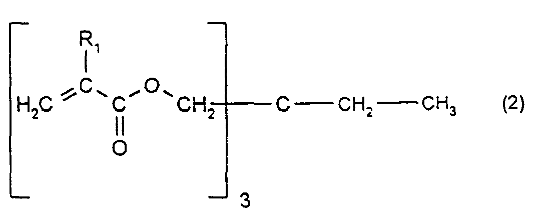 dans laquelle R₁ désigne un atome d'hydrogène ou un alkyle en C₁-C₄ et plus particulièrement méthyle (triméthylol propane triacrylate).
De manière préférée, on choisit ces polymères réticulés parmi ceux présentant une viscosité mesurée au viscosimètre BROOKFIELD, mobile 4, à une vitesse de rotation de 100 tours/minutes dans une solution d'eau à 2 % et à 25 °C supérieure ou égale à 1000 centipoises et plus préférentiellement allant de 5000 à 40000 centipoises et plus particulièrement de 6500 à 35000 centipoises.
Ces polymères sont notamment décrits dans la demande EP815844.
Il est à noter que cette demande EP815844 est relative à une composition cosmétique ou dermatologique sous forme d'émulsion huile-dans-eau, qui est stable tout en ne contenant pas de tensioactif, et qui comprend par ailleurs un polymère poly(acide 2-acrylamido 2-méthylpropane sulfonique) réticulé et neutralisé à au moins 90%. Ce polymère est ajouté à la phase aqueuse, qu'il épaissit. Toutefois, dans la composition ainsi obtenue, la phase grasse ne peut représenter que jusqu'à environ 12-15% en poids de l'émulsion. Au-delà de cette quantité de phase grasse, la composition perd en stabilité.

Les copolymères constitués d'une fraction majoritaire de monomère acide carboxylique monooléfiniquement insaturé en C₃-C₆ ou de son anhydride, et d'une fraction minoritaire de monomère ester à chaîne grasse d'acide acrylique, susceptibles d'être utilisés dans le cadre de la présente invention, éventuellement réticulés, peuvent être préparés en polymérisant une quantité prépondérante de monomère carboxylique monooléfiniquement insaturé ou de son anhydride, avec une quantité plus faible de monomère ester acrylique à chaîne grasse. La quantité de monomère carboxylique ou de son anhydride, est de préférence comprise entre 80 et 98% en poids et plus particulièrement entre 90 et 98% en poids; l'ester acrylique est de préférence présent dans des quantités comprises entre 2 et 20% en poids et plus particulièrement entre 2 et 10% en poids; les pourcentages sont calculés par rapport au poids des deux monomères.
Les monomères carboxyliques préférentiels sont choisis parmi ceux répondant à la formule :

CH₂=CR-COOH

dans laquelle R désigne hydrogène, halogène, hydroxyle, un groupe lactone, un groupe lactame, un groupe cyanogène (-CN), un groupe alkyle monovalent, un groupe aryle, un groupe alkylaryle, un groupe aralkyle ou un groupe cycloaliphatique.

Les monomères carboxyliques particulièrement préférés, sont choisis parmi l'acide acrylique, l'acide méthacrylique, l'anhydride maléique, et leurs mélanges.
Les monomères esters acryliques à chaîne grasse sont préférentiellement choisis parmi ceux répondant à la formule: CH₂=CR¹-COOR ²
dans laquelle R¹ est choisi dans le groupe formé par hydrogène, méthyle et éthyle et R² est un groupe alkyle en C₈-C₃₀, un groupe oxyalkylène en C₈-C₃₀, un groupe carbonyloxyalkylène en C₈-C₃₀.
Les monomères esters particulièrement préférés sont ceux pour lesquels R¹ est hydrogène ou méthyle, et/ou ceux pour lesquels R² est un groupe alkyle en C₁₀₋C₂₂. On peut notamment citer les acrylates et méthacrylates de décyle, de lauryle, de stéaryle, de béhényle ou de mélissyle.
Certains de ces copolymères sont notamment décrits dans la demande EP-A-0268164 et sont obtenus selon les méthodes de préparation décrites dans ce même document.
On peut citer plus particulièrement les copolymères vendus sous le nom PEMULEN par la Société GOODRICH, et notamment le copolymère acrylate/C₁₀₋C₃₀-alkylacrylate tel que le produit PEMULEN TR 2.

Dans le cadre de la présente invention, on peut, bien évidemment, utiliser un mélange de plusieurs épaississants hydrophiles tels que ci-dessus définis.
Lesdits épaississants hydrophiles peuvent être présents dans les compositions cosmétiques ou dermatologiques de l'invention dans des concentrations allant de préférence de 0,05-10 % en poids par rapport au poids total de la composition et plus préférentiellement de 0,5-4 % en poids.
De préférence, la viscosité de la phase aqueuse est de l'ordre de 30 000 à 80 000 centipoises (30-80 Pa.s) mesurée au viscosimètre Brookfield, aiguille 7 à une vitesse de 20 tours/minute.

L'émulsion selon l'invention comprend par ailleurs une phase huileuse qui comprend au moins un copolymère épaississant choisi parmi les copolymères comprenant au moins un motif hydrophobe dans une quantité suffisante pour obtenir leur solubilité partielle ou totale dans ladite phase huileuse, et au moins un motif hydrophile dans une quantité suffisante pour produire un épaississement de ladite phase huileuse; ledit motif hydrophile étant choisi parmi les monoacides carboxyliques à insaturation α,β-éthylénique en C₃-C₆, les diacides carboxyliques à insaturation α,β-éthylénique en C₄-C₆ ainsi que les dérivés monesters ou monoamides desdits diacides.
Cet épaississant peut notamment être choisi parmi ceux décrits et préparés dans le brevet US 5,736,125.
Le ou les motifs hydrophobes dudit copolymère épaississant peuvent être constitués par des (méth)acrylates d'alkyle en C₁₀-C₂₂; des (méth)acrylamides d'alkyle en C₁₀-C₂₂ ; les esters et éthers vinyliques en C₁₀-C₂₂ ; des siloxanes; des α-oléfines en C₁₀-C₂₂; des chaînes aliphatiques latérales d'au moins 6 atomes de carbone fluorées; des chaînes aliphatiques latérales d'alkyl(C₁-C₂₄) styrène d'au moins 6 atomes de carbone et plus particulièrement par les (méth)acrytates en C₁₈-C₂₂. Le ou les motifs hydrophobes représentent en général de 80 à 98% en poids et de préférence de 85 à 97% du poids total du copolymère épaississant.
Le ou les motifs hydrophiles dudit copolymère épaississant, nécessaires pour épaissir la phase huileuse, peuvent être constitués par l'acide acrylique, l'acide (méth)-acrylique; l'acide maléique ou l'acide itaconique ou leurs monoesters ou monoamides d'alcools en C₁-C₂₂ et plus particulièrement par l'acide acrylique et/ou l'acide méthacrylique.
Les copolymères épaississants lipophiles conformes à l'invention ont en général un indice d'acide allant de 0,1 à 4,0 meq/g et plus particulièrement de 0,4 à 2 meq/g. Leur poids moléculaire moyen est d'au moins 50000 daltons et varie de préférence de 50000 à 200000 daltons.
Les copolymères épaississants lipophiles particulièrement préférés sont choisis parmi les copolymères (méth)acrylate d'alkyle en C₁₀-C₂₂/acide (méth)acrylique dans lesquels la quantité de (méth)acrylate d'alkyle est suffisante pour obtenir la solubilité partielle ou totale dudit polymère dans une phase huileuse et la quantité d'acide (méth)acrylique est suffisante pour épaissir la phase huileuse.
Ils sont encore plus particulièrement choisis parmi :
- les copolymères acrylate de docosyle/styrène/acide acrylique dans lesquels la quantité d'acrylate de docosyle et de styrène est suffisante pour obtenir une solubilité partielle ou totale dudit polymère dans une phase huileuse et la quantité d'acide acrylique est suffisante pour épaissir la phase huileuse, tels que les produits décrits sous les noms SAMPLE 124-93 (72/4/2% en poids), SAMPLE 124-130 (68/27/5% en poids), SAMPLE 108-195 (67/28/5% en poids) dans le brevet US 5,736,125 et fabriqués par la Société LANDEC CORPORATION ; et/ou
- les copolymères acrylate de stéarylelacide méthacrylique dans lesquels la quantité d'acrylate de stéaryle est suffisante pour obtenir une solubilité partielle ou totale dudit polymère dans une phase huileuse et la quantité d'acide méthacrylique est suffisante pour épaissir la phase huileuse, tels que les produits décrits sous les noms SAMPLE 124-194 (92,5/7,5% en poids), SAMPLE 124-195 (90/10% en poids) dans le brevet US 5,736,125 et fabriqués par la Société LANDEC CORPORATION.
Dans le cadre de la présente invention, on peut, bien évidemment, utiliser un mélange de plusieurs copolymères épaississants lipophiles tels que ci-dessus définis.

Lesdits copolymères épaississants lipophiles peuvent être présents dans les compositions cosmétiques ou dermatologiques de l'invention dans des concentrations allant de préférence de 0,1-10 % en poids par rapport au poids total de la composition et plus préférentiellement de 0,5-2 % en poids.

De préférence, la viscosité de la phase huileuse est de l'ordre de 6 000 à 20 000 centipoises (6-20 Pa.s) mesurée au viscosimètre Brookfield, aiguille 5 à une vitesse de 20 tours/minute.

La phase grasse de l'émulsion peut comprendre toute huile susceptible d'être utilisée dans le domaine d'application considéré. En effet, un des avantages relatif à l'utilisation du copolymère épaississant lipophile ci-dessus est qu'il permet d'épaissir toute huile, quelle que soit sa nature chimique.
Par huile, on entend toute matière grasse liquide à température ambiante (25°C).
La phase grasse peut donc comprendre une ou plusieurs huiles choisies de préférence parmi :
- les huiles de silicone, volatiles ou non, linéaires, ramifiées ou cycliques, éventuellement organomodifiées; les silicones phénylées; les résines et les gommes de silicone liquides à température ambiante;
- les huiles minérales telles que l'huile de paraffine et de vaseline,
- les huiles d'origine animale telles que le perhydrosqualène, la lanoline;
- les huiles d'origine végétale telles que les triglycérides liquides, par exemple les huiles de tournesol, de maïs, de soja, de jojoba, de courge, de pépins de raisin, de sésame, de noisette, d'abricot, de macadamia, d'avocat, d'amande douce, de ricin, les triglycérides des acides caprylique/caprique comme ceux vendus par la société STEARINERIES DUBOIS ou ceux vendus sous les dénominations MIGLYOL 810, 812 et 818® par la société DYNAMO NOBEL; l'huile d'olive, l'huile d'arachide, l'huile de colza, l'huile de coprah;
- les huiles de synthèse telles que l'huile de purcellin, les isoparaffines; les alcools gras; les esters d'acides gras;
- les huiles fluorées et perfluorées,
- les huiles de silicones fluorées;
- leurs mélanges.
L'émulsion selon l'invention peut comprendre 1-50% en poids de phase grasse, de préférence 5-30 % en poids, et plus préférentiellement 10-20% en poids de phase grasse.

De façon connue, l'émulsion selon l'invention peut éventuellement contenir une faible quantité d'un tensioactif notamment H/E, bien que cela ne soit pas nécessaire pour obtenir une émulsion stable. La quantité de tensioactif peut représenter de 0,1 à 3% et de préférence de 0,1 à 2 % du poids total de l'émulsion. Avantageusement, l'émulsion ne comprend pas de tensioactif.

L'émulsion selon l'invention peut être notamment utilisée dans les domaines cosmétique et/ou dermatologique. Elle peut être utilisée telle quelle, et donc constituer elle-même une composition cosmétique ou dermatologique; elle peut également être incorporée dans une composition cosmétique ou dermatologique plus élaborée.

Les compositions selon l'invention, constituées par ou comprenant ladite émulsion, contiennent un milieu cosmétiquement ou dermatologiquement acceptable, c'est-à-dire un milieu compatible avec toutes les matières kératiniques telles que la peau, les ongles, les muqueuses et les cheveux ou toute autre zone cutanée du corps. Ce milieu peut comprendre, de manière connue en soi, les constituants usuellement employés dans le domaine d'application considéré.
En particulier, ces compositions peuvent comprendre :
- des cires choisies parmi les cires animales, fossiles, végétales, minérales ou de synthèse connues en soi, telles que les cires de paraffine, les cires de polyéthylène, les cires de Carnauba, de Candellila; les cires d'abeilles; la cire microcristalline; les cires de silicone.
- des solvants organiques;
- des actifs cosmétiques et/ou dermatologiques, hydrophiles ou lipophiles, tels que les adoucissants, les antioxydants, les opacifiants, les émollients, les hydroxyacides, les agents anti-mousse, les hydratants, les vitamines, les parfums, les conservateurs, les colorants, les séquestrants, les polymères, les propulseurs, les agents alcalinisants ou acidifiants, des filtres UV, des céramides; des agents anti-radicaux libres; des agents amincissants ; des bactéricides; des antipelliculaires; des complexants; des absorbeurs d'odeur;
- des matières pulvérulentes telles que des charges, des pigments et/ou des nacres.
Ces adjuvants, selon leur nature, peuvent être introduits dans la phase grasse ou dans la phase aqueuse. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

L'émulsion et la composition selon l'invention peuvent être préparées selon les techniques connues de l'homme du métier.
En particulier, l'émulsion peut être préparée en solubilisant l'épaississant lipophile dans la phase grasse chauffée à 60-80°C, puis en solubilisant l'épaississant hydrophile dans la phase aqueuse chauffée à 60-80°C, et en dispersant ladite phase grasse dans ladite phase aqueuse sous agitation.

Les compositions selon l'invention peuvent se présenter sous forme de suspension ou de dispersion dans des corps gras; sous forme de dispersion vésiculaire non ionique; sous forme d'émulsion simple ou complexe (H/E, E/H, H/E/H ou E/H/E), de préférence de type huile-dans-eau; une crème, un lait, un gel, une pommade, une mousse aérosol ou un spray; un sérum, une pâte.
Elles trouvent en particulier une application dans un grand nombre de traitements cosmétique ou dermatologique de la peau, des cheveux, des cils, des sourcils, des ongles, des muqueuses, du cuir chevelu; en particulier on peut citer le soin du visage et/ou du corps (crèmes de protection, de traitement ou de soin pour le visage, pour les mains ou pour le corps; laits corporels de protection ou de soin; lotions, gels ou mousses pour le soin de la peau et des muqueuses ou pour le nettoyage de la peau); le maquillage du visage et/ou du corps (rouge à lèvres, eye-liner, fond de teint, mascara, anti-cernes, fard à paupières ou à joues); le démaquillage; la protection solaire; le traitement dermatologique des maladies de la peau, des cheveux, des cils des sourcils, des ongles, du cuir chevelu et/ou des muqueuses.
Elles trouvent une application préférée dans des compositions, cosmétique ou dermatologique, destinées aux peaux sèches et/ou sensibles.

L'invention est illustrée plus en détail dans les exemples suivants, dans lesquels les % sont donnés en poids.

### Exemple 1: Crème nutritive pour peau très sèche

| *phase A* | |
|---|---|
| Poly(acide 2-acrylamido 2-méthylpropane sulfonique) réticulé | |
| (exemple A de EP815844) à 2% dans l'eau | 1,5% |
| Glycérol | 5 % |
| Conservateurs | 0,3 % |
| Eau | qsp 100 % |

| *phase B* | |
|---|---|
| Huile d'amandes d'abricots | 25 % |
| Isoparaffine hydrogénée | 10 % |
| Copolymère acrylate de stéaryle/acide méthacrylique (92,5/7,5% en poids) correspondant à 'SAMPLE 124-194' dans le brevet US 5,736,125 | 1 % |

L'émulsion est préparée de la manière suivante : les phases A et B sont préparées par simple mélange à chaud des constituants et homogénéisées séparément sous agitation à 70-75°C. Puis on disperse sous agitation la phase grasse B dans la phase aqueuse A.

On obtient une émulsion fine et régulière, dont les bords sont nets. L'émulsion reste stable pendant au moins deux mois, à une température de 20°C et à une température de 37°C.

### Exemple 2: Crème riche pour le soin du corps

| *phase A* | |
|---|---|
| Poly(acide 2-acrylamido 2-méthylpropane sulfonique) réticulé (exemple A de EP815844) à 2% dans l'eau | 1 % |
| Glycérol | 5 % |
| Conservateurs | 0,3 % |
| Eau | qsp 100 % |

| *phase B* | |
|---|---|
| Huile de vaseline | 15 % |
| Myristate de myristyle | 10 % |
| Copolymère acrylate de stéaryle/acide méthacrylique (92,517,5% en poids) correspondant à 'SAMPLE 124-194' dans le brevet US 5,736,125 | 1 % |

L'émulsion est préparée selon l'exemple 1. On obtient une émulsion fine, souple et facile à appliquer. Elle est douce et nourrissante.
Elle présente une bonne stabilité à 20°C, 37°C et 45°C pendant au moins deux mois.

### Exemple 3: Fluide démaquillant visage pour peaux sensibles

| *phase A* | |
|---|---|
| Copolymère acrylate/C₁₀-C₃₀-alkylacrylate (PEMULEN TR 2) | 0,2 % |
| Conservateurs | 0,3% |
| Eau | qsp 100% |

| *phase B* | |
|---|---|
| Palmitate d'octyle | 20 % |
| Copolymère acrylate de stéarylelacide méthacrylique (92,517,5% en poids) correspondant à'SAMPLE 124-194' dans le brevet US 5,736,125 | 0,5% |

L'émulsion est préparée selon l'exemple 1. On obtient une émulsion souple, légère, présentant un très bon pouvoir démaquillant sans agresser la peau.

### Exemple 4

On compare, au microscope, les émulsions suivantes :
- émulsion A selon l'exemple 1
- émulsion B : émulsion selon l'exemple 1 sans copolymère acrylate de stéaryle/acide méthacrylique.
- émulsion C : émulsion selon l'exemple 1 dans laquelle on a remplacé le copolymère acrylate de stéaryle/acide méthacrylique par du stéarate de glycérol.

On obtient les résultats suivants :
- l'émulsion A est fine, régulière, les bords sont nets.
- l'émulsion B est grossière au microscope. Elle est instable et se casse le jour même de la fabrication. On obtient une mauvaise stabilité notamment parce que le taux de phase grasse est trop important. A partir de 15% environ de phase grasse liquide, le poly(acide 2-acrylamido 2-méthylpropane sulfonique) réticulé, connu pour stabiliser les émulsions, ne suffit plus pour avoir une émulsion stable.
- l'émulsion C est très grossière au microscope, les bords sont très "lâches". Elle est instable et se 'déphase' rapidement. L'émulsion glisse sur les parois du flacon, ce qui démontre également son instabilité : elle est huileuse. De plus, les propriétés cosmétiques de l'émulsion C sont inférieures à celles de l'émulsion A : texture moins lisse, grasse, pas fraîche.

## Revendications

1. Emulsion comprenant une phase aqueuse et une phase huileuse, et comprenant en outre au moins un composé épaississant hydrophile et au moins un copolymère épaississant comprenant au moins un motif hydrophobe dans une quantité suffisante pour obtenir sa solubilité partielle ou totale dans ladite phase huileuse, et au moins un motif hydrophile dans une quantité suffisante pour produire un épaississement de ladite phase huileuse; ledit motif hydrophile étant choisi parmi les monoacides carboxyliques à insaturation α,β-ethylénique en C₃₋C₆, les diacides carboxyliques à insaturation α,β-éthylénique en C₄-C₆ ainsi que les dérivés monesters ou monoamides desdits diacides.

2. Emulsion selon la revendication 1, dans laquelle le ou les motifs hydrophobes du copolymère épaississant sont constitués par des (méth)acrylates d'alkyle en C₁₀-C₂₂; des (méth)acrylamides d'alkyle en C₁₀-C₂₂; les esters et éthers vinyliques en C₁₀-C₂₂ ; des siloxanes ; des α-oléfines en C₁₀-C₂₂ ; des chaînes aliphatiques latérales d'au moins 6 atomes de carbone fluorées; des chaînes aliphatiques latérales d'alkyl(C₁-C₂₄) styrène d'au moins 6 atomes de carbone et plus particulièrement par les (méth)acrylates en C₁₈-C₂₂.

3. Emulsion selon l'une des revendications précédentes, dans laquelle le ou les motifs hydrophobes du copolymère épaississant représentent 80 à 98% en poids, de préférence 85 à 97% du poids total dudit copolymère épaississant.

4. Emulsion selon l'une des revendications précédentes, dans laquelle le ou les motifs hydrophiles du copolymère épaississant sont constitués par l'acide acrylique, l'acide (méth)-acrylique; l'acide maléique ou l'acide itaconique ou leurs monoesters ou monoamides d'alcools en C₁-C₂₂ et plus particulièrement par l'acide acrylique et/ou l'acide méthacrylique.

5. Emulsion selon l'une des revendications précédentes, dans laquelle les copolymères épaississants sont choisis parmi les copolymères (méth)acrylate d'alkyle en C₁₀-C₂₂/acide (méth)acrylique, et en particulier parmi les copolymères acrylate de docosyle/styrène/acide acrylique et/ou les copolymères acrylate de stéaryle/acide méthacrylique.

6. Emulsion selon l'une des revendications précédentes, dans laquelle le copolymère épaississant est présent à une concentration allant de préférence de 0,1-10 % en poids par rapport au poids total de la composition et plus préférentiellement de 0,5-2 % en poids.

7. Emulsion selon l'une des revendications précédentes, dans laquelle le composé épaississant hydrophile est choisi parmi :
- les polymères synthétiques,
- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose,
- le silicate de magnésium et d'aluminium;
- les épaississants inorganiques tels que les smectites, les hectorites modifiées ou non (BENTONE, LAPONITE par exemple),
- leurs mélanges.

8. Emulsion selon l'une des revendications précédentes, dans laquelle le composé épaississant hydrophile est choisi parmi:
(A) les acides polyacryliques et notamment les polymères poly(méth)acrylates de glycéryl; les homopolymères et les copolymères d'acide acrylique, éventuellement réticulés, ou un de leurs sels,
(B) les polymères à base de polyacrylamide et notamment :
(i) une émulsion H/E comprenant 35-45% en poids de copolymère réticulé d'acrylamide/acide 2-acrylamido 2-méthylpropane sulfonique neutralisé, 15-25% en poids d'hydrocarbures isoparaffiniques, 3-8% en poids de lauryléther de polyéthylèneglycol 7OE, et d'eau;
(ii) les copolymères acrylate/octyl-acrylamide ;
(iii) les polymères réticulés d'acrylamide et de chlorure de méthacryloyloxyéthyltriméthylammonium;
(iv) les polymères réticulés d'acrylamide et d'acrylate d'ammonium ;
(v) les polymères poly(acide 2-acrylamido 2-méthylpropane sulfonique) réticulés et pratiquement ou totalement neutralisés;
(C) les copolymères constitués d'une fraction majoritaire de monomère acide carboxylique monooléfiniquement insaturé en C₃-C₆ ou de son anhydride, et d'une fraction minoritaire de monomère ester à chaîne grasse d'acide acrylique, ces copolymères étant éventuellement réticulés.

9. Emulsion selon l'une des revendications précédentes, dans laquelle le composé épaississant hydrophile est choisi parmi les polymères poly(acide 2-acrylamido 2-méthylpropane sulfonique) réticulés et pratiquement ou totalement neutralisés, comprenant, distribués de façon aléatoire:
a) de 90 à 99,9% en poids de motifs de formule générale (1) suivante : 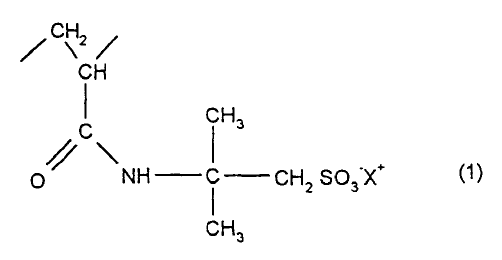 dans laquelle X⁺ désigne un cation ou un mélange de cations, au plus 10% mol des cations X⁺ pouvant être des protons H⁺ ;
b) de 0,01 à 10% en poids de motifs réticulants provenant d'au moins un monomère ayant au moins deux doubles liaisons oléfiniques ; les proportions en poids étant définis par rapport au poids total du polymère.

10. Emulsion selon l'une des revendications précédentes, dans laquelle le composé épaississant hydrophile est un copolymère acrylate/C₁₀-C₃₀-alkylacrylate.

11. Emulsion selon l'une des revendications précédentes, dans laquelle le composé épaississant hydrophile est présent à une concentration de 0,05-10 % en poids, préférentiellement de 0,5-4 % en poids.

12. Emulsion selon l'une des revendications précédentes, se présentant sous la forme d'une émulsion huile-dans-eau, comprenant une phase grasse ou huileuse interne dispersée dans une phase externe aqueuse.

13. Emulsion selon l'une des revendications précédentes, dans laquelle la phase huileuse comprend une ou plusieurs huiles choisies parmi :
- les huiles de silicone, volatiles ou non, linéaires, ramifiées ou cycliques, éventuellement organomodifiées; les silicones phénylées; les résines et les gommes de silicone liquides à température ambiante;
- les huiles minérales telles que l'huile de paraffine et de vaseline,
- les huiles d'origine animale telles que le perhydrosqualène, la lanoline;
- les huiles d'origine végétale telles que les triglycérides liquides, par exemple les huiles de tournesol, de maïs, de soja, de jojoba, de courge, de pépins de raisin, de sésame, de noisette, d'abricot, de macadamia, d'avocat, d'amande douce, de ricin, les triglycérides des acides caprylique/caprique; l'huile d'olive, l'huile d'arachide, l'huile de colza, l'huile de coprah;
- les huiles de synthèse telles que l'huile de purcellin, les isoparaffines; les alcools gras; les esters d'acides gras;
- les huiles fluorées et perfluorées,
- les huiles de silicones fluorées;
- leurs mélanges.

14. Emulsion selon l'une des revendications précédentes, comprenant 1-50% en poids de phase grasse, de préférence 5-30 % en poids, et plus préférentiellement 10-20% en poids de phase grasse.

15. Emulsion selon l'une des revendications précédentes, ne comprenant pas de tensioactif.

16. Composition cosmétique ou dermatologique comprenant, dans un milieu cosmétiquement ou dermatologiquement acceptable, une émulsion telle que définie selon l'une des revendications 1 à 15.

17. Utilisation d'une émulsion telle que définie selon l'une des revendications 1 à 15, pour le traitement cosmétique de la peau, des cheveux, des cils, des sourcils, des ongles, des muqueuses, du cuir chevelu; en particulier pour le soin du visage et/ou du corps, le maquillage du visage et/ou du corps, le démaquillage; la protection solaire.

18. Utilisation d'une émulsion telle que définie selon l'une des revendications 1 à 15, pour la fabrication d'une composition dermatologique destinée au traitement de la peau, des cheveux, des cils, des sourcils, des ongles, du cuir chevelu et/ou des muqueuses.

19. Utilisation selon l'une des revendications 17 à 18, destinée aux peaux sèches et/ou sensibles.

20. Procédé de traitement non thérapeutique de la peau, des cheveux, des cils, des sourcils, des ongles, des muqueuses, du cuir chevelu, consistant à appliquer sur le support une émulsion telle que définie selon l'une des revendications 1 à 15, ou une composition telle que définie selon la revendication 16.

21. Procédé de traitement non thérapeutique des peaux sèches et/ou sensibles, consistant à appliquer sur le support une émulsion telle que définie selon l'une des revendications 1 à 15, ou une composition telle que définie selon la revendication 16.

22. Utilisation d'un composé épaississant hydrophile et d'un copolymère épaississant comprenant au moins un motif hydrophobe dans une quantité suffisante pour obtenir sa solubilité partielle ou totale dans ladite phase huileuse, et au moins un motif hydrophile dans une quantité suffisante pour produire un épaississement de ladite phase huileuse; ledit motif hydrophile étant choisi parmi les monoacides carboxyliques à insaturation α,β-éthylénique en C₃-C₆, les diacides carboxyliques à insaturation α,β-éthylénique en C₄-C₆ ainsi que les dérivés monesters ou monoamides desdits diacides, pour stabiliser une émulsion.

23. Utilisation selon la revendication 22, pour stabiliser une émulsion huile-dans-eau.

24. Utilisation selon l'une des revendications 22 à 23, pour stabiliser une émulsion ne comprenant pas de tensioactif.

## Claims

1. Emulsion comprising an aqueous phase and an oily phase and additionally comprising at least one hydrophilic thickening compound and at least one thickening copolymer comprising at least one hydrophobic unit in an amount sufficient to obtain its partial or complete solubility in the said oily phase and at least one hydrophilic unit in an amount sufficient to produce thickening of the said oily phase; the said hydrophilic unit being chosen from C₃-C₆ monocarboxylic acids with α,β-ethylenic unsaturation, C₄-C₆ dicarboxylic acids with α,β-ethylenic unsaturation, and the monoester or monoamide derivatives of the said diacids.

2. Emulsion according to Claim 1, in which the hydrophobic unit or units of the thickening copolymer are composed of C₁₀-C₂₂ alkyl (meth)acrylates; (C₁₀-C₂₂ alkyl)(meth)acrylamides; C₁₀-C₂₂ vinyl esters and ethers; siloxanes; C₁₀-C₂₂ α-olefins; fluorinated aliphatic side chains with at least 6 carbon atoms; or aliphatic side chains of (C₁-C₂₄ alkyl) styrene with at least 6 carbon atoms and more particularly of C₁₈-C₂₂ (meth)acrylates.

3. Emulsion according to either of the preceding claims, in which the hydrophobic unit or units of the thickening copolymer represent 80 to 98% by weight, preferably 85 to 97%, of the total weight of the said thickening copolymer.

4. Emulsion according to one of the preceding claims, in which the hydrophilic unit or units of the thickening copolymer are composed of acrylic acid, methacrylic acid, maleic acid or itaconic acid or their monoesters or monoamides with C₁-C₂₂ alcohols and more particularly of acrylic acid and/or methacrylic acid.

5. Emulsion according to one of the preceding claims, in which the thickening copolymers are chosen from C₁₀-C₂₂ alkyl (meth)acrylate/(meth)-acrylic acid copolymers and in particular from docosyl acrylate/styrene/acrylic acid copolymers and/or stearyl acrylate/methacrylic acid copolymers.

6. Emulsion according to one of the preceding claims, in which the thickening copolymer is present at a concentration preferably ranging from 0.1 to 10% by weight with respect to the total weight of the composition and more preferably from 0.5 to 2% by weight.

7. Emulsion according to one of the preceding claims, in which the hydrophilic thickening compound is chosen from:
- synthetic polymers,
- polysaccharide biopolymers, such as xanthan gum, locust bean gum, guar gum, alginates or modified celluloses, such as hydroxyethylcellulose, methylcellulose, hydroxypropylcellulose and carboxymethylcellulose,
- aluminium magnesium silicate;
- inorganic thickeners, such as smectites or hectorites, whether modified or unmodified (Bentone or Laponite, for example),
- their mixtures.

8. Emulsion according to one of the preceding claims, in which the hydrophilic thickening compound is chosen from:
(A) polyacrylic acids and in particular poly(glyceryl (meth)acrylate) polymers; or optionally crosslinked acrylic acid homopolymers and copolymers, or one of their salts,
(B) polyacrylamide-based polymers and in particular:
(i) an O/W emulsion comprising 35-45% by weight of crosslinked neutralized acrylamide/2-acrylamido-2-methylpropanesulphonic acid copolymer, 15-25% by weight of isoparaffin hydrocarbons, 3-8% by weight of polyethylene glycol 7EO lauryl ether, and water;
(ii) acrylate/octylacrylamide copolymers;
(iii) crosslinked polymers of acrylamide and of methacryloyloxyethyltrimethylammonium chloride;
(iv) crosslinked polymers of acrylamide and of ammonium acrylate;
(v) crosslinked and virtually or completely neutralized poly(2-acrylamido-2-methylpropanesulphonic acid) polymers;
(C) copolymers composed of a major fraction of monoolefinically unsaturated C₃-C₆ carboxylic acid monomer or of its anhydride and of a minor fraction of ester with a fatty chain of acrylic acid monomer, these copolymers optionally being crosslinked.

9. Emulsion according to one of the preceding claims, in which the hydrophilic thickening compound is chosen from crosslinked and virtually or completely neutralized poly(2-acrylamido-2-methyl-propanesulphonic acid) polymers comprising, distributed randomly:
a) from 90 to 99.9% by weight of units of following general formula (1): 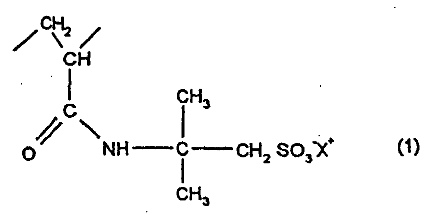 in which X⁺ denotes a cation or a mixture of cations, at most 10 mol% of the X⁺ cations being able to be H⁺ protons;
b) from 0.01 to 10% by weight of crosslinking units originating from at least one monomer having at least two olefinic double bonds; the proportions by weight being defined with respect to the total weight of the polymer.

10. Emulsion according to one of the preceding claims, in which the hydrophilic thickening compound is an acrylate/C₁₀-C₃₀ alkyl acrylate copolymer.

11. Emulsion according to one of the preceding claims, in which the hydrophilic thickening compound is present at a concentration of 0.05 to 10% by weight, preferably of 0.5 to 4% by weight.

12. Emulsion according to one of the preceding claims, which is provided in the form of an oil-in-water emulsion comprising an internal fatty or oily phase dispersed in an external aqueous phase.

13. Emulsion according to one of the preceding claims, in which the oily phase comprises one or more oils chosen from:
- optionally organomodified, volatile or nonvolatile, linear, branched or cyclic silicone oils; phenylated silicones; or silicone resins and gums which are liquid at room temperature;
- mineral oils, such as liquid paraffin and liquid petrolatum,
- oils of animal origin, such as perhydrosqualene or lanolin;
- oils of vegetable origin, such as liquid triglycerides, for example sunflower, maize, soybean, jojoba, cucumber, grape seed, sesame, hazelnut, apricot, macadamia, avocado, sweet almond or castor oils, triglycerides of caprylic/capric acids, olive oil, groundnut oil, rapeseed oil or coconut oil;
- synthetic oils, such as purcellin oil, isoparaffins, fatty alcohols or fatty acid esters;
- fluorinated and perfluorinated oils;
- fluorinated silicone oils;
- their mixtures.

14. Emulsion according to one of the preceding claims, comprising 1 to 50% by weight of fatty phase, preferably 5 to 30% by weight and more preferably 10 to 20% by weight of fatty phase.

15. Emulsion according to one of the preceding claims, not comprising surfactant.

16. Cosmetic or dermatological composition comprising, in a cosmetically or dermatologically acceptable medium, an emulsion as defined according to one of Claims 1 to 15.

17. Use of an emulsion as defined according to one of Claims 1 to 15 for the cosmetic treatment of the skin, hair, eyelashes, eyebrows, nails, mucous membranes or scalp; in particular for caring for the face and/or body, making up the face and/or body, removing make-up, or antisun protection.

18. Use of an emulsion as defined according to one of Claims 1 to 15 in the manufacture of a dermatological composition intended for the treatment of the skin, hair, eyelashes, eyebrows, nails, scalp and/or mucous membranes.

19. Use according to either of Claims 17 and 18, intended for dry and/or sensitive skin.

20. Process for the nontherapeutic treatment of the skin, hair, eyelashes, eyebrows, nails, mucous membranes or scalp which consists in applying, to the substrate, an emulsion as defined according to one of Claims 1 to 15 or a composition as defined according to Claim 16.

21. Process for the nontherapeutic treatment of dry and/or sensitive skin which consists in applying, to the substrate, an emulsion as defined according to one of Claims 1 to 15 or a composition as defined according to Claim 16.

22. Use of a hydrophilic thickening compound and of a thickening copolymer comprising at least one hydrophobic unit in an amount sufficient to obtain its partial or complete solubility in the said oily phase and at least one hydrophilic unit in an amount sufficient to produce thickening of the said oily phase; the said hydrophilic unit being chosen from C₃-C₆ monocarboxylic acids with α,β-ethylenic unsaturation, C₄-C₆ dicarboxylic acids with α,β-ethylenic unsaturation, and the monoester or monoamide derivatives of the said diacids, for stabilizing an emulsion.

23. Use according to Claim 22, for stabilizing an oil-in-water emulsion.

24. Use according to either of Claims 22 and 23, for stabilizing an emulsion not comprising surfactant.

## Patentansprüche

1. Emulsion, die eine wässrige Phase und eine Ölphase und ferner mindestens eine hydrophile verdickende Verbindung und mindestens ein verdickendes Copolymer enthält, das mindestens eine hydrophobe Einheit in einer Menge, die ausreichend ist, um in der Ölphase eine teilweise oder vollständige Solubilisierung zu erzielen, und mindestens eine hydrophile Einheit in einer Menge enthält, die ausreichend ist, damit eine Verdickung der Ölphase auftritt; wobei die hydrophile Einheit unter den α,β-ethylenisch ungesättigten Monocarbonsäuren mit 3 bis 6 Kohlenstoffatomen, α,β-ethylenisch ungesättigten Dicarbonsäuren mit 4 bis 6 Kohlenstoffatomen sowie den Monoester- oder Monoamidderivaten dieser Disäuren ausgewählt ist.

2. Emulsion nach Anspruch 1, wobei die hydrophobe(n) Einheit(en) des verdickenden Copolymers aus C₁₀-₂₂-Alkyl(meth)acrylaten; C₁₀₋₂₂-Alkyl(meth)acrylamiden; C₁₀₋₂₂-Vinylestern und -ethern; Siloxanen; C₁₀₋₂₂-α-Olefinen; aliphatischen Seitenketten mit mindestens 6 fluorierten Kohlenstoffatomen; aliphatischen Seitenketten von Alkyl(C₁₋₂₄)styrol mit mindestens 6 Kohlenstoffatomen und insbesondere den (Meth)acrylaten mit 18 bis 22 Kohlenstoff atomen bestehen.

3. Emulsion nach einem der vorhergehenden Ansprüche, bei der die hydrophobe(n) Einheit(en) des verdickenden Copolymers 80 bis 98 Gew.-% und vorzugsweise 85 bis 97 Gew.% des Gesamtgewichts des verdickenden Copolymers ausmachen.

4. Emulsion nach einem der vorhergehenden Ansprüche, bei der die hydrophile(n) Einheit(en) des verdickenden Copolymers aus Acrylsäure, Methacrylsäure; Maleinsäure oder Itaconsäure oder deren Monoestern oder Monoamiden von C₁₋₂₂-Alkoholen und insbesondere Acrylsäure und/oder Methacrylsäure bestehen.

5. Emulsion nach einem der vorhergehenden Ansprüche, wobei die verdickenden Copolymere unter den C₁₀₋₂₂-Alkyl(meth)acrylat/(Meth)acrylsäure-Copolymeren und insbesondere den Docosylacrylat/Styrol/Acrylsäure-Copolymeren und/oder Stearylacrylat/(Meth)acrysäure-Copolymeren ausgewählt sind.

6. Emulsion nach einem der vorhergehenden Ansprüche, wobei das verdickende Copolymer in einer Konzentration von vorzugsweise 0,1 bis 10 Gew.-%, bezogen auf das Gesamtgewicht der Zusammensetzung, und noch bevorzugter 0,5 bis 2 Gew.-% enthalten ist.

7. Emulsion nach einem der vorhergehenden Ansprüche, wobei die hydrophile verdickende Verbindung ausgewählt ist unter:
- synthetischen Polymeren,
- Polysaccharidbiopolymeren, wie Xanthangummi, Johannisbrot-Kernmehl, Guargummi, Alginaten, modifizierten Cellulosen, wie Hydroxyethylcellulose, Methylcellulose, Hydroxypropylcellulose und Carboxymethylcellulose,
- Magnesiumaluminiumsilicat;
- anorganischen Verdickungsmitteln, wie Smectiten, modifizierten oder nicht modifizierten Hectoriten (beispielsweise BENTONE, LAPONITE),
- und deren Gemischen.

8. Emulsion nach einem der vorhergehenden Ansprüche, bei der die hydrophile verdickende Verbindung ausgewählt ist unter:
(A) Polyacrylsäuren und insbesondere Polyglyceryl(meth)acrylatpolymeren und Homopolymeren und Copolymeren von Acrylsäuren, die gegebenenfalls vernetzt sind, oder einem ihrer Salze;
(B) Polymeren auf der Basis von Polyacrylamid und insbesondere:
(i) einer O/W-Emulsion, die 35 bis 45 Gew.-% vernetztes Acrylamid/neutralisierte 2-Acrylamido-2-methylpropansulfonsäure-Copolymer, 15 bis 25 Gew.-% isoparaffinische Kohlenwasserstoffe, 3 bis 8 Gew.-% Polyethylenglykollaurylether 7 EO und Wasser enthält;
(ii) Acrylat/Octylacrylamid-Copolymeren;
(iii) vernetzten Polymeren von Acrylamid und Methacryloyloxyethyltrimethylammoniumchlorid;
(iv) vernetzten Polymeren von Acrylmid und Ammoniumacrylat;
(v) Poly-(2-acrylamido-2-methylpropansulfonsäure)-Polymeren, die vernetzt und ganz oder teilweise neutralisiert sind;
(C) Copolymeren, die hauptsächlich aus einem monoolefinisch ungesättigten Carbonsäuremonomer mit 3 bis 6 Kohlenstoffatomen oder seinem Anhydrid und in einem geringeren Anteil einem Acrylsäureestermonomer mit Fettkette bestehen, wobei diese Copolymere gegebenenfalls vernetzt sind.

9. Emulsion nach einem der vorhergehenden Ansprüche, wobei die hydrophile verdickende Verbindung unter den vernetzten und ganz oder teilweise neutralisierten 2-(Acrylamido-2-methyl-propansulfonsäure)-Polymeren ausgewählt ist, die zufällig verteilt enthalten:
a) 90 bis 99,9 Gew.-% Einheiten der folgenden allgemeinen Formel (1): 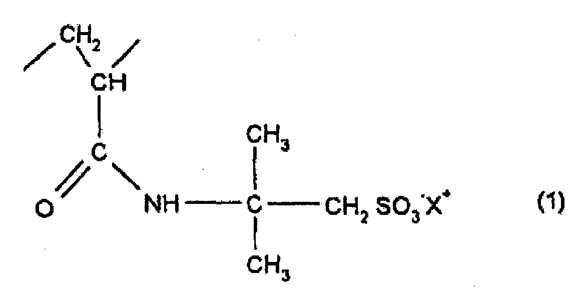 worin X⁺ ein Kation oder ein Gemisch von Kationen bedeutet, wobei höchstens 10 Mol-% der Kationen X⁺ Protonen H⁺ sein können;
b) 0,01 bis 10 Gew.-% vernetzende Einheiten, die von mindestens einem Monomer mit mindestens zwei olefinischen Doppelbindungen abgeleitet sind; wobei die Gewichtsanteile bezogen auf das Gesamtgewicht des Polymers definiert sind.

10. Emulsion nach einem der vorhergehenden Ansprüche, wobei die hydrophile verdickende Verbindung ein Acrylat/C₁₀-₃₀-Alkylacrylat-Copolymer ist.

11. Emulsion nach einem der vorhergehenden Ansprüche, wobei die hydrophile verdickende Verbindung in einer Konzentration von 0,05 bis 10 Gew.-% und vorzugsweise 0,5 bis 4 Gew.-% enthalten ist.

12. Emulsion nach einem der vorhergehenden Ansprüche, die als Ölin-Wasser-Emulsion vorliegt, die eine in einer äußeren wässrigen Phase dispergierte innere Fettphase oder Ölphase enthält.

13. Emulsion nach einem der vorhergehenden Ansprüche, **dadurch gekennzeichnet, dass** die Ölphase ein oder mehrere Öle enthält, die ausgewählt sind unter:
- flüchtigen oder nichtflüchtigen, linearen, verzweigten oder cyclischen, gegebenenfalls organomodifizierten Siliconölen; phenylierten Siliconen; Siliconharzen und Silicongummis, die bei Umgebungstemperatur flüssig sind;
- Mineralölen, wie Paraffinöl und Vaselineöl;
- Ölen tierischer Herkunft, wie Perhydrosqualen, Lanolin;
- Ölen pflanzlicher Herkunft, wie flüssigen Triglyceriden, beispielsweise Sonnenblumenöl, Maisöl, Sojaöl, Jojobaöl, Kürbiskernöl, Traubenkernöl, Sesamöl, Haselnussöl, Aprikosenkernöl, Macadamiaöl, Avocadoöl, Süßmandelöl, Ricinusöl, Triglyceriden von Capryl/ Caprinsäure; Olivenöl, Erdnussöl, Rapsöl, Kopraöl;
- synthetischen Ölen, wie Purcellinöl, Isoparaffinen, Fettalkoholen; Fettsäureestern;
- fluorierten und perfluorierten Ölen;
- fluorierten Siliconölen; und
- deren Gemischen.

14. Emulsion nach einem der vorhergehenden Ansprüche, die 1 bis 50 Gew.-% Fettphase, vorzugsweise 5 bis 30 Gew.-% und noch bevorzugter 10 bis 20 Gew.-% Fettphase enthält.

15. Emulsion nach einem der vorhergehenden Ansprüche, die keinen grenzflächenaktiven Stoff enthält.

16. Kosmetische oder dermatologische Zusammensetzung, die in einem kosmetisch oder dermatologisch akzeptablen Medium eine Emulsion nach einem der Ansprüche 1 bis 15 enthält.

17. Verwendung einer Emulsion nach einem der Ansprüche 1 bis 15 für die kosmetische Behandlung der Haut, der Haare, der Wimpern, der Augenbrauen, der Nägel, der Schleimhäute, der Kopfhaut; insbesondere für die Pflege des Gesichts und/oder des Körpers, zum Schminken des Gesichts und/oder des Körpers, zum Abschminken; für den Sonnenschutz.

18. Verwendung einer Emulsion nach einem der Ansprüche 1 bis 15 für die Herstellung einer dermatologischen Zusammensetzung, die für die Behandlung der Haut, der Haare, der Wimpern, der Augenbrauen, der Nägel, der Kopfhaut und/oder der Schleimhäute vorgesehen ist.

19. Verwendung nach einem der Ansprüche 17 bis 18 für trockene und/oder empfindliche Hauttypen.

20. Verfahren zur nichttherapeutischen Behandlung der Haut, der Haare, der Wimpern, der Augenbrauen, der Nägel, der Schleimhäute, der Kopfhaut, das darin besteht, auf den Träger eine Emulsion nach einem der Ansprüche 1 bis 15 oder eine Zusammensetzung nach Anspruch 16 aufzutragen.

21. Verfahren zur nichttherapeutischen Behandlung von trockener und/oder empfindlicher Haut, das darin besteht, auf den Träger eine Emulsion nach einem der Ansprüche 1 bis 15 oder eine Zusammensetzung nach Anspruch 16 aufzubringen.

22. Verwendung einer hydrophilen verdickenden Verbindung und eines verdickendes Copolymers, das mindestens eine hydrophobe Einheit in einer Menge, die für die partielle oder vollständige Solubilisierung in der Ölphase ausreichend ist, und mindestens eine hydrophile Einheit in einer Menge enthält, die für eine Verdickung der Ölphase ausreichend ist, wobei die hydrophile Einheit unter den α,β-ethylenisch ungesättigten C₃₋₆-Monocarbonsäuren, α,β-ethylenisch ungesättigten C₄₋₆-Dicarbonsäuren sowie den Monoestern oder Monoamiden dieser Disäuren ausgewählt ist, für die Stabilisierung einer Emulsion.

23. Verwendung nach Anspruch 22 zur Stabilisierung einer Öl-in-Wasser-Emulsion.

24. Verwendung nach einem der Ansprüche 22 bis 23 zur Stabilisierung einer Emulsion, die keine grenzflächenaktiven Stoffe enthält.
